(19) **Europäisches Patentamt / European Patent Office / Office européen des brevets**

(11) **EP 1 530 946 B1**

(12) **EUROPEAN PATENT SPECIFICATION**

(45) Date of publication and mention
of the grant of the patent:
**08.04.2009 Bulletin 2009/15**

(51) Int Cl.:
***A61B 5/053*** (2006.01)

(21) Application number: **04257084.6**

(22) Date of filing: **16.11.2004**

(54) **Human skin impedance model representing a skin impedance response at high frequency**

Hautimpedanzmodell zur Darstellung der Hautimpedanz bei hohen Frequenzen

Modèle de l'impédance de la peau réprésentant l'impédance de la peau à haute fréquence

(84) Designated Contracting States:
**DE FR GB**

(30) Priority: **17.11.2003 KR 2003081101**

(43) Date of publication of application:
**18.05.2005 Bulletin 2005/20**

(73) Proprietor: **SAMSUNG ELECTRONICS CO., LTD.
Suwon-si,
Gyeonggi-do (KR)**

(72) Inventors:
• **Jang, Woo-young**
  **Seoul (KR)**
• **Cho, Jin-ho**
  **School of E.E.&C.S., Kyungpook Nat. U.
  Daegu-si (KR)**
• **Shin, Sang-hoon**
  **Bundgang-gu, Seongnam-si
  Gyeonggi-do (KR)**
• **Lee, Jeong-Woo**
  **School of E.E.&C.S.
  Buk-gu
  Daegu-si (KR)**

(74) Representative: **Anderson, James Edward George et al
Elkington and Fife LLP
Prospect House
8 Pembroke Road
Sevenoaks, Kent TN13 1XR (GB)**

(56) References cited:
• **LEE J-W ET AL: "CHARACTERISTICS OF HUMAN SKIN IMPEDANCE INCLUDING AT BIOLOGICAL ACTIVE POINTS" IEICE TRANSACTIONS ON FUNDAMENTALS OF ELECTRONICS, COMMUNICATIONS AND COMPUTER SCIENCES, INSTITUTE OF ELECTRONICS INFORMATION AND COMM. ENG. TOKYO, JP, vol. E86A, no. 6, June 2003 (2003-06), pages 1476-1479, XP001171088 ISSN: 0916-8508**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

The present invention relates to an analysis of constituents of a body. More particularly, the present invention relates to a human skin impedance model representing a skin impedance response in a high frequency band.

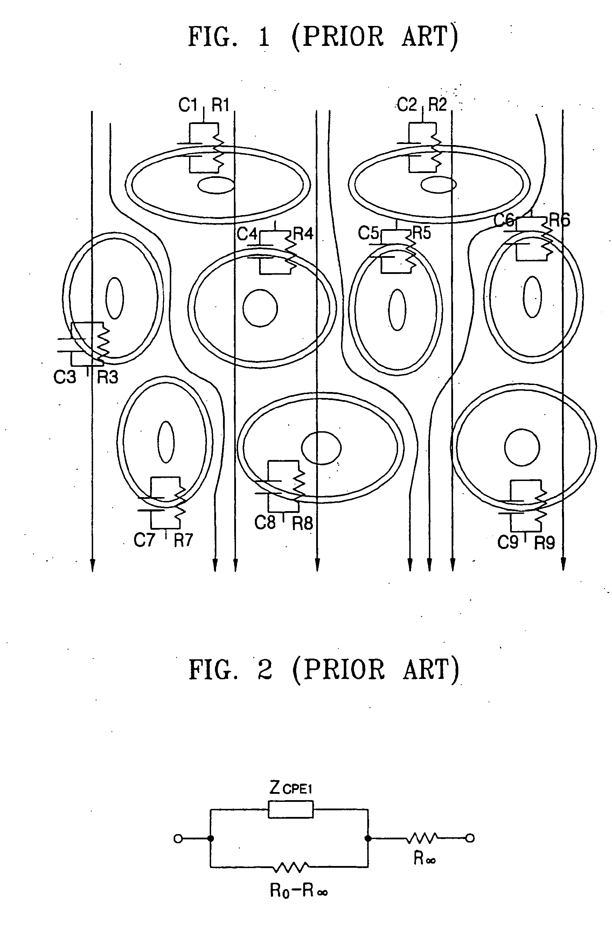

By measuring a body electric impedance (hereinafter referred to as "impedance") in a human body, a condition of skin may be estimated, an amount of body fat may be measured, a degree of medicine penetration on skin may be measured, or a response of skin to stimulation may be examined. FIG. 1 is a diagram for explaining a structure of a cell of a living body using an equivalent electric circuit. Referring to FIG. 1, due to variations in a shape and size of each cell, it may be seen that when each cell is expressed as an RC equivalent circuit, there is a slight difference among time constants (RC). More specifically, a first cell may have first time constants R1 and C1, a second cell may have second time constants R2 and C2, etc.

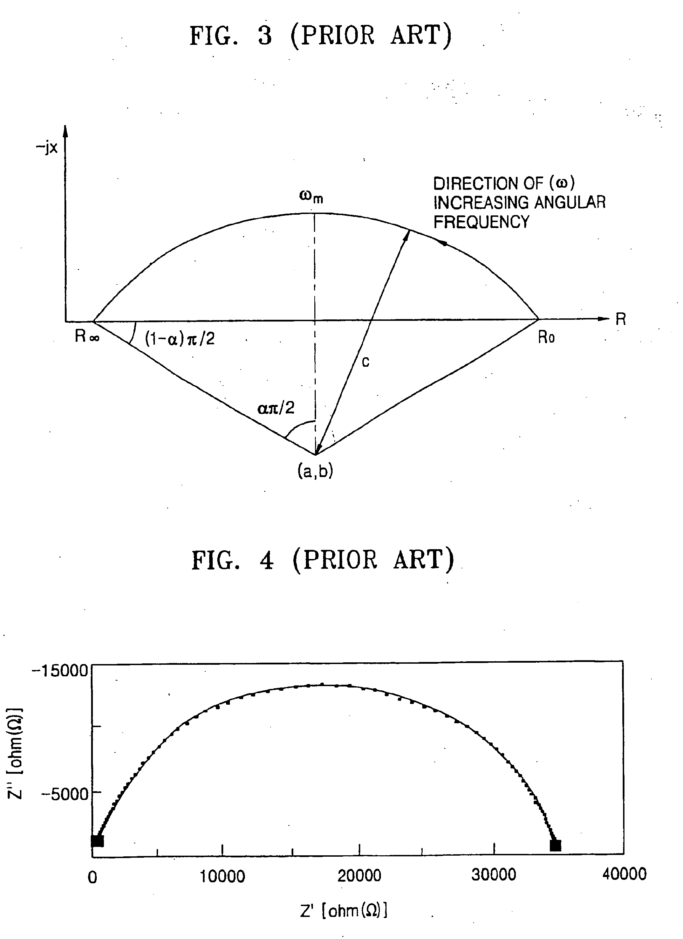

FIG. 2 is a diagram showing a conventional skin impedance model proposed by Cole. FIG. 3 is a diagram showing a locus of living body impedance represented on a complex impedance locus by the Cole impedance model. When a frequency is low, the impedance is located on $R_0$ on a complex impedance plane, and as the angular frequency ($\omega$) increases, the impedance traces a semicircular locus. Finally, at a high frequency, the impedance converges on $R_\infty$.

The Cole impedance model uses a device, called a constant phase element (CPE), together with resistors. The CPE is a device having a characteristic in a middle between a resistor and a capacitor, and can be expressed as the following equation 1:

$$Z_{CPE} = k(j\omega)^{-\alpha} ......(1)$$

where k denotes the amplitude of the constant phase element (CPE) at angular frequency $\omega = 1$ rad/s and $\alpha$ denotes a property between a property of a resistor and a property of a capacitor, e.g., 0.5-1 in the case of human skin.

A formula for the Cole model including the CPE device can be expressed as the following equation 2:

$$Z = R_\infty + \frac{R_0 - R_\infty}{1 + (R_0 - R_\infty)(Z_{CPE})^{-1}} = R_\infty + \frac{R_0 - R_\infty}{1 + \dfrac{R_0 - R_\infty}{k}(j\omega)^\alpha} ......(2)$$

Equation 2 is used as a basic model for living body impedance.

If a result of a simulation of the impedance values from 1 Hz to 10 kHz, after appropriate parameters for the Cole impedance model are set, is represented on a complex impedance plane, the impedance locus as shown in FIG. 4 is obtained.

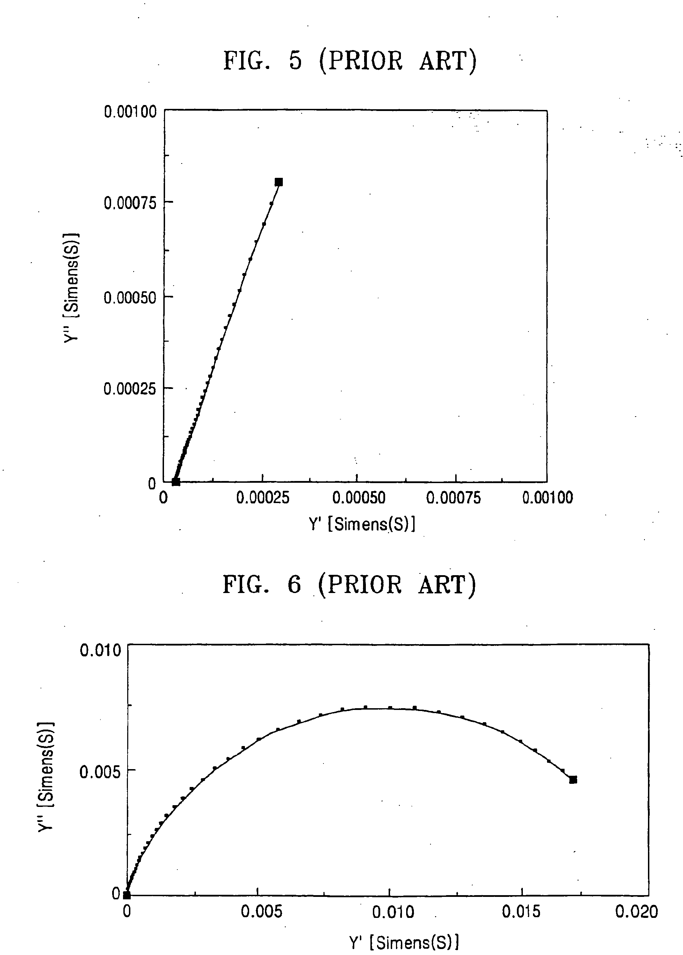

Since the impedance converges on $R\infty$ at frequencies of 10 kHz or above, it is difficult to represent the impedance data on a complex impedance plane. In order to observe skin impedance characteristics at a high frequency band, e.g., over tens of kHz, the data can be represented on a complex admittance plane. If data from 1 Hz to 10 kHz are represented on a complex admittance plane, a graph similar to a straight line as shown in FIG. 5 is obtained, which does not form a complete semi-circular locus.

If skin impedance is represented on a complex admittance plane after simulating up to 2 MHz using the Cole impedance model, a semicircle locus as shown in FIG. 6 is obtained. Accordingly, in order to observe the characteristics of skin impedance at a high frequency area, measured data or simulation results should be represented on a complex admittance plane.

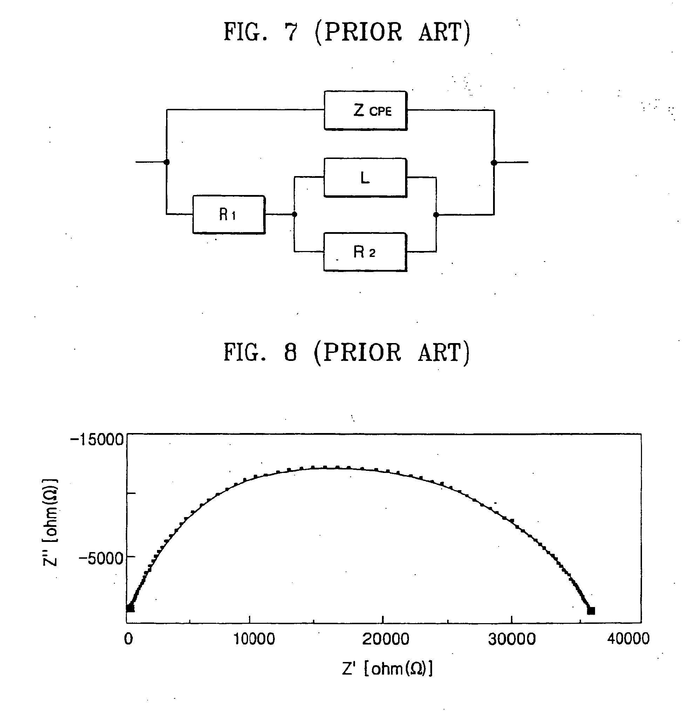

Among research on living body impedance, research on the impedance characteristics of skin, which is a specific part of a living body, is also being actively conducted. Among them, another conventional skin impedance model proposed by Kontturi, in which the skin impedance is modeled as electric devices, is shown in FIG. 7. The skin impedance model of FIG. 7 can be expressed as the following equation 3:

$$Z_{Kontturi} = \frac{kR_1 R_2 (j\omega)^{-\alpha} + kL(R_1 + R_2)(j\omega)^{1-\alpha}}{R_1 R_2 + j\omega L(R_1 + R_2) + kR_2 j\omega^{-\alpha} + kL(j\omega)^{1-\alpha}} ......(3)$$

As described above, when measured skin impedance data are represented on a complex impedance plane, the locus in a low frequency area does not form a complete semicircle but a round locus with an end of the locus partially opened. To solve this problem, Kontturi improved the characteristics of the skin impedance response by adding resistances and inductance to the existing Cole impedance model.

Since a maximum value of the measured frequency used in the Kontturi skin impedance model is 10 kHz, characteristics of skin impedance at higher frequencies are not considered.

A result of a simulation using the Kontturi skin impedance model represented on a complex impedance plane is shown in FIG 8. The graph of FIG. 8 does not show a complete locus but shows a shape in which the low frequency area is partially opened. Accordingly, the Kontturi skin impedance model accurately represents the skin impedance response at a low frequency area.

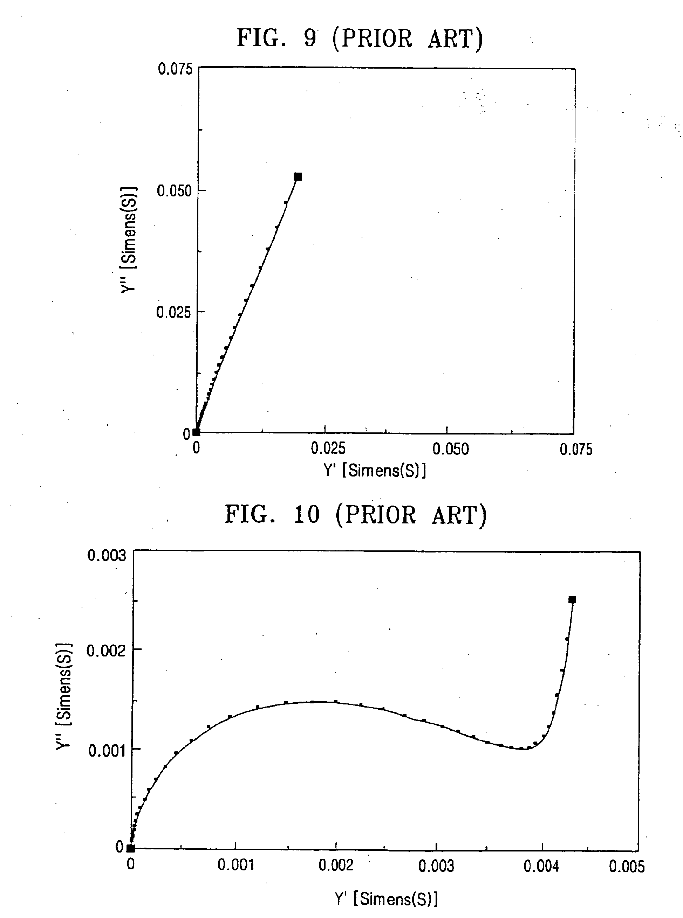

However, if the response to 2 MHz is obtained through simulation, it is represented on a complex impedance plane, as in FIG. 8, such that the characteristics at the high frequency area cannot be identified. If the simulation result is represented on a complex admittance plane, a graph similar to a straight line as shown in FIG. 9 is obtained.

Actually, if in order to identify the frequency response of skin, skin impedance data up to 2 MHz are measured and represented on a complex admittance plane, it is shown as in FIG. 10 that admittance increases in a high frequency band of tens of MHz.

When FIGS. 9 and 10 are compared, it may be seen that the Kontturi impedance model is also unable to accurately represent the skin response at a high frequency band.

Lee J. W. et al: "Characteristics of Human Skin Impedance Including at Biological Active Points", IEICE Transactions on Fundamentals of Electronics, Communications and Computer Sciences, Institute of Electronics Information and Comm. Eng., Tokyo, vol. E86A, no. 6, June 2003, pp. 1476-1479, discloses a skin impedance model for biological active or acupuncture points. The model for these points comprises an equivalent electrical circuit with two Cole impedance models in series.

According to the present invention, there is provided a method of analyzing skin impedance of a predetermined part of a living body, the method comprising: providing a predetermined current between a first terminal and a third terminal on the predetermined part; obtaining skin impedance data by measuring a voltage between a second terminal on the predetermined part and the third terminal, the second terminal being located between the first terminal and the third terminal; and providing a skin impedance model for between the second terminal and the third terminal using the skin impedance data, the model comprising: a first area having a first resistor and a first constant phase element connected in parallel; a second area having a second resistor and a second constant phase element connected in parallel, and a third resistor serially connected to the parallel connection of the second resistor and the second constant phase element; and a third area having a third constant phase element, wherein the second area and the third area are connected in parallel and are serially connected to the first area.

The skin impedance model may be expressed as:

$$Z = \frac{R_1 k_1 (j\omega)^{-a1}}{R_1 + k_1 (j\omega)^{-a1}} + \frac{R_2 R_3 R_4 k_2 (j\omega)^{-a2} + R_2 (R_3 + R_4) k_2 k_3 (j\omega)^{-(a2+a3)}}{R_2 R_3 R_4 + (R_2 R_3 + R_3 R_4) k_2 (j\omega)^{-a2} + R_2 (R_3 + R_4) k_2 k_3 (j\omega)^{-a3} + (R_2 + R_3 + R_4) k_2 k_3 (j\omega)^{-(a2+a3)}} \ .$$

The present invention also provides a computer program comprising computer program code means adapted to perform the method described above when said program is run on a computer.

The present invention thus provides a skin impedance model representing characteristics of a skin response at a high frequency band, e.g., on the order of megahertz (MHz).

The above and other features and advantages of the present invention will become more apparent to those of ordinary skill in the art by describing in detail preferred embodiments thereof with reference to the attached drawings in which:

FIG. 1 is a diagram for explaining a structure of a cell of a living body using equivalent circuit diagrams;
FIG. 2 is a diagram showing a conventional skin impedance model proposed by Cole;
FIG. 3 is a diagram showing a locus of living body impedance represented on a complex impedance locus by the Cole impedance model;
FIG. 4 is a diagram showing a result of a simulation of impedance values from 1 Hz to 10 kHz using the Cole impedance model, represented on a complex impedance plane;
FIG. 5 is a diagram showing data from 1 Hz to 10 kHz obtained using the Cole impedance model, represented on a complex admittance plane;
FIG. 6 is a diagram showing a result of a simulation up to a 2 MHz band using the Cole impedance model, represented on a complex admittance plane;
FIG. 7 is a diagram showing another conventional skin impedance model proposed by Kontturi;

FIG. 8 is a diagram showing a result of a simulation using the Kontturi skin impedance model, represented on a complex impedance plane;

FIG. 9 is a diagram showing a result of a simulation from 1 Hz to 2 MHz using the Kontturi skin impedance model, represented on a complex admittance plane;

FIG. 10 is a diagram of skin impedance data from 1 Hz to 2 MHz measured in order to test a frequency response of skin, represented on a complex admittance plane;

FIG. 11 is a diagram illustrating a skin impedance model according to a first embodiment of the present invention;

FIG. 12 is a diagram illustrating a 3-electrode method for measuring a skin impedance;

FIG. 13 is a table showing skin impedance data measured by the 3-electrode method;

FIG. 14 is a diagram illustrating a skin impedance model according to a second embodiment of the present invention; and

FIG. 15 is a diagram illustrating a skin impedance model according to a third embodiment of the present invention.

The present invention will now be described more fully hereinafter with reference to the accompanying drawings, in which preferred embodiments of the invention are shown. The invention may, however, be embodied in different forms and should not be construed as limited to the embodiments set forth herein. Rather, these embodiments are provided so that this disclosure will be thorough and complete, and will fully convey the scope of the invention to those skilled in the art. Like reference numerals and characters refer to like elements throughout.

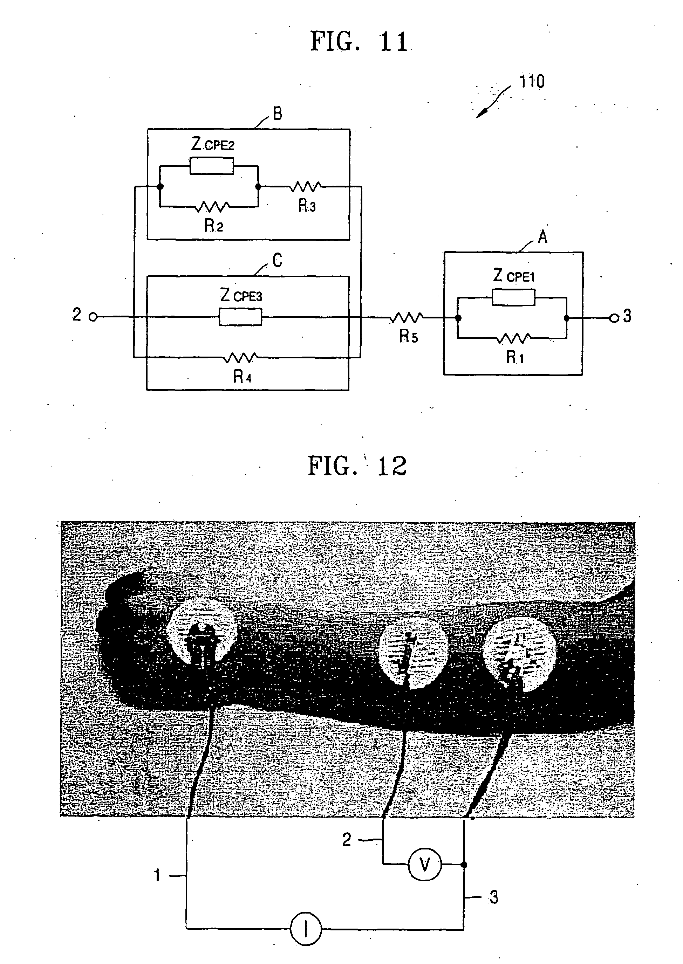

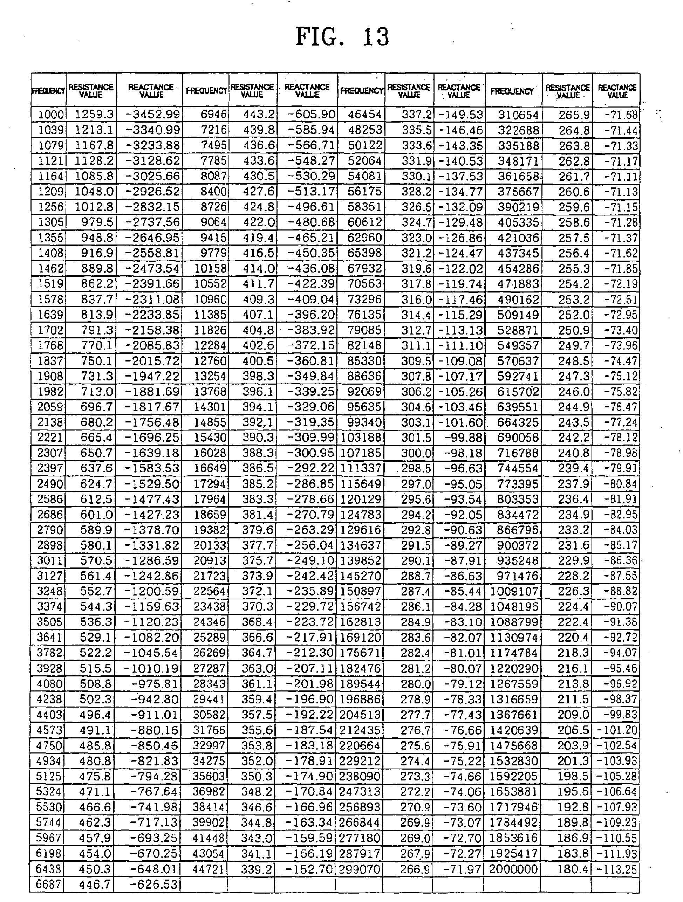

FIG. 11 is a diagram illustrating a skin impedance model according to a first embodiment of the present invention, and is obtained by analyzing skin impedance data measured using a 3-electrode method as illustrated in FIG. 12. In the 3-electrode method, by providing a predetermined current (I) between a first terminal 1 and a third terminal 3, a voltage (V) between a second terminal 2 and the third terminal 3 may be measured. FIG. 13 is a table showing skin impedance data of a forearm part on thirty-six (36) frequency spots at a frequency band from 1 kHz to 2 MHz.

The skin impedance model 110 between second and third terminals 2 and 3 of FIG. 11 obtained from the skin impedance data of FIG. 13 is represented by a first area A, a second area B, and a third area C. The first area A is a portion representing an impedance of outer skin where a first resistor $R_1$ and a first CPE $Z_{CPE1}$ are connected in parallel. The second area B is a portion representing a membrane and an intercellular fluid in corium constituents of skin, in which a second resistor $R_2$ and a second CPE $Z_{CPE2}$ are connected in parallel, and then serially connected to a third resistor $R_3$. The third area C is a portion representing an intercellular fluid in corium constituents of skin, in which a fourth resistor $R_4$ and a third CPE $Z_{CPE3}$ are connected in parallel. The second area B and the third area C are connected in parallel and then serially connected to the first area A through a fifth resistor $R_5$. This skin impedance model 110 may be expressed as the following equation 4:

$$Z = \frac{R_1 k_1 (j\omega)^{-a1}}{R_1 + k_1 (j\omega)^{-a1}} + \frac{R_2 R_3 R_4 k_2 (j\omega)^{-a2} + R_2 (R_3 + R_4) k_2 k_3 (j\omega)^{-(a2+a3)}}{R_2 R_3 R_4 + (R_2 R_3 + R_3 R_4) k_2 (j\omega)^{-a2} + R_2 (R_3 + R_4) k_2 k_3 (j\omega)^{-a3} + (R_2 + R_3 + R_4) k_2 k_3 (j\omega)^{-(a2+a3)}} \quad \ldots\ldots(4)$$

Parameters and errors applied to the skin impedance model 110 are as shown in Table 1:

Table 1

|  |  | Parameters |
|---|---|---|
| $R_4$ |  | 444.1 |
| $Z_{CPE3}$ | T | 5.0357E-10 |
|  | P | 0.94037 |
| $R_2$ |  | 2700 |
| $Z_{CPE2}$ | T | 9.2921 E-08 |
|  | P | 0.74856 |
| $R_3$ |  | 610.5 |
| $R_5$ |  | 3E-07 |
| $R_1$ |  | 22397 |
| $Z_{CPE1}$ | T | 7.0978E-08 |
|  | P | 0.94386 |

(continued)

|  | Parameters |
|---|---|
| $X^2$ ERROR | 3.954E-6 |

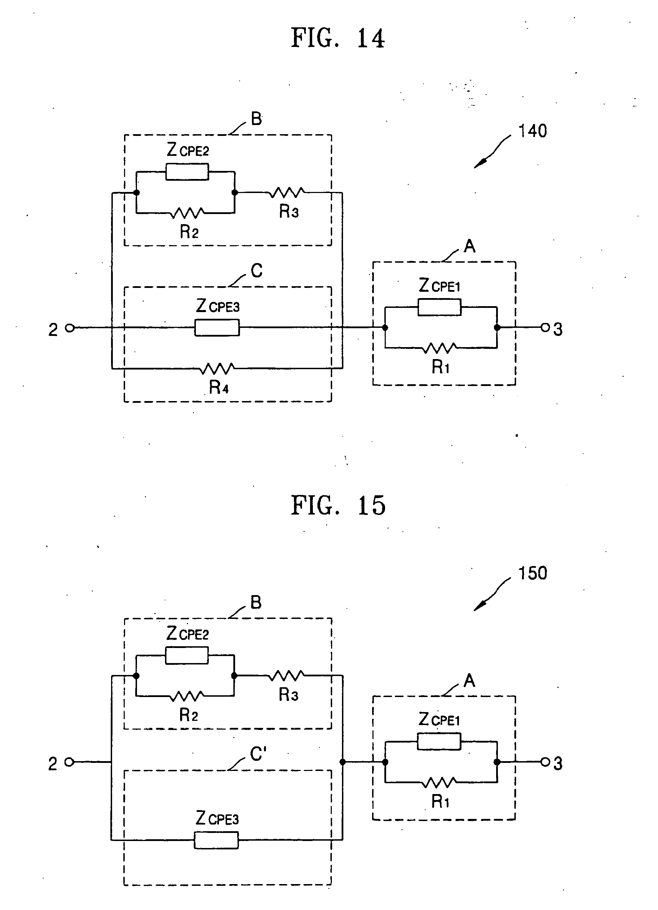

FIG. 14 is a diagram for explaining a skin impedance model according to a second embodiment of the present invention. Referring to FIG. 14, the skin impedance model 140 includes a first area A where a first resistor $R_1$ is connected to a first CPE $Z_{CPE1}$ in parallel, a second area B where a second resistor $R_2$ and a second CPE $Z_{CPE2}$ are connected in parallel and then are serially connected to a third resistor $R_3$, and a third area 3 where a fourth resistor $R_4$ and a third CPE $Z_{CPE3}$ are connected in parallel. The second area B and the third area C are connected in parallel and then are serially connected to the first area A. The skin impedance model 140 of the second embodiment of the present invention is a simplified model as compared to the model of the first embodiment and may be obtained by removing the fifth resistor $R_5$ from the skin impedance model 110 according to the first embodiment as shown in FIG. 11.

Parameters and errors applied to the skin impedance model 140 are as shown in Table 2:

Table 2

|  |  | Parameters |
|---|---|---|
| $R_4$ |  | 445 |
| $Z_{CPE3}$ | T | 5.0357E-10 |
|  | P | 0.94037 |
| $R_2$ |  | 2651 |
| $Z_{CPE2}$ | T | 9.3456E-08 |
|  | P | 0.74856 |
| $R_3$ |  | 608.7 |
| $R_1$ |  | 22397 |
| $Z_{CPE1}$ | T | 7.0978E-08 |
|  | P | 0.94386 |
| $X^2$ ERROR |  | 3.954E-6 |

FIG. 15 is a diagram for explaining a skin impedance model according to a third embodiment of the present invention. Referring to FIG. 15, the skin impedance model 150 includes a first area A where a first resistor $R_1$ is connected to a first CPE $Z_{CPE1}$ in parallel, a second area B where a second resistor $R_2$ and a second CPE $Z_{CPE2}$ are connected in parallel and then are serially connected to a third resistor $R_3$, and a third area C' having a third CPE $Z_{CPE3}$. The second area B and the third area C' are connected in parallel and then are serially connected to the first area A. The skin impedance model 150 of the third embodiment of the present invention is the most simplified model as compared to the models of the first and second embodiments and may be obtained by including the characteristics of the fourth resistor $R_4$ in the second resistor $R_2$ and the third resistor $R_3$ from the skin impedance model 140 according to the second embodiment of the present invention as shown in FIG. 14.

Parameters and errors applied to the skin impedance model 150 are as shown in Table 3:

Table 3

|  |  | Parameters |
|---|---|---|
| $Z_{CPE3}$ | T | 5.0357E-10 |
|  | P | 0.94037 |
| $R_2$ |  | 134.5 |
| $Z_{CPE2}$ | T | 5.2404E-07 |
|  | P | 0.74856 |
| $R_3$ |  | 257.1 |

(continued)

|  |  | Parameters |
|---|---|---|
| $R_1$ |  | 22397 |
| $Z_{CPE1}$ | T | 7.0978E-08 |
|  | P | 0.94386 |
| $X^2$ ERROR |  | 3.9338E-6 |

Accordingly, it may be seen that in the skin impedance models according to the embodiments of the present invention, small $x^2$ errors of about 4E-6 occur and the measured skin impedance data of FIG. 13 are accurately represented.

Preferred embodiments of the present invention have been disclosed herein and, although specific terms are employed, they are used and are to be interpreted in a generic and descriptive sense only and not for purpose of limitation. Accordingly, it will be understood by those of ordinary skill in the art that various changes in form and details may be made without departing from the scope of the present invention as set forth in the following claims.

## Claims

1. A method of analyzing skin impedance of a predetermined part of a living body, the method comprising:

   providing a predetermined current between a first terminal (1) and a third terminal (3) on the predetermined part;
   obtaining skin impedance data by measuring a voltage between a second terminal (2) on the predetermined part and the third terminal (3), the second terminal (2) being located between the first terminal (1) and the third terminal (3); and
   providing a skin impedance model for between the second terminal (2) and the third terminal (3) using the skin impedance data, the model comprising:

   a first area (A) having a first resistor and a first constant phase element connected in parallel;
   a second area (B) having a second resistor and a second constant phase element connected in parallel, and a third resistor serially connected to the parallel connection of the second resistor and the second constant phase element; and
   a third area having a third constant phase element,
   wherein the second area and the third area are connected in parallel and are serially connected to the first area.

2. The method of claim 1, wherein the third area (C) has a fourth resistor connected in parallel with the third constant phase element.

3. The method of claim 1, wherein the second area (B) and the third area (C) are connected in parallel and are serially connected to the first area (A) through a fifth resistor.

4. The method as claimed in any preceding claim, wherein the skin impedance model is expressed as:

$$ Z = \frac{R_1 k_1 (j\omega)^{-a1}}{R_1 + k_1 (j\omega)^{-a1}} + \frac{R_2 R_3 R_4 k_2 (j\omega)^{-a2} + R_2 (R_3 + R_4) k_2 k_3 (j\omega)^{-(a2+a3)}}{R_2 R_3 R_4 + (R_2 R_3 + R_3 R_4) k_2 (j\omega)^{-a2} + R_2 (R_3 + R_4) k_2 k_3 (j\omega)^{-a3} + (R_2 + R_3 + R_4) k_2 k_3 (j\omega)^{-(a2+a3)}} . $$

5. A computer program comprising computer program code means adapted to perform the method of any preceding claim when said program is run on a computer.

6. A computer program as claimed in claim 5 embodied on a computer readable medium.

**Patentansprüche**

1. Verfahren zum Analysieren der Hautimpedanz eines vorgegebenen Teils eines lebenden Körpers, wobei das Verfahren umfasst:

   Bereitstellen eines vorgegebenen Stroms zwischen einem ersten Anschluss (1) und einem dritten Anschluss (3) auf dem vorgegebenen Körperteil,
   Ermitteln von Hautimpedanzdaten durch Messen einer Spannung zwischen einem zweiten Anschluss (2) auf dem vorgegebenen Körperteil und dem dritten Anschluss (3), wobei der zweite Anschluss (2) zwischen dem ersten Anschluss (1) und dem dritten Anschluss (3) gelegen ist, und
   Bereitstellen eines Hautimpedanzmodells zwischen dem zweiten Anschluss (2) und dem dritten Anschluss (3) unter Verwendung der Hautimpedanzdaten, wobei das Modell umfasst:

   einen ersten Bereich (A) mit einem ersten Widerstand und einem parallel geschalteten ersten Element mit konstanter Phase,
   einen zweiten Bereich (B) mit einem zweiten Widerstand und einem parallel geschalteten zweiten Element mit konstanter Phase und einem dritten Widerstand, der mit der Parallelschaltung des zweiten Widerstands und des zweiten Elements mit konstanter Phase in Serie geschaltet ist, und
   einen dritten Bereich mit einem dritten Element mit konstanter Phase,
   wobei der zweite Bereich und der dritte Bereich parallel geschaltet sind und mit dem ersten Bereich in Serie geschaltet sind.

2. Verfahren nach Anspruch 1, wobei der dritte Bereich (C) einen vierten Widerstand aufweist, der mit dem dritten Element mit konstanter Phase parallel geschaltet ist.

3. Verfahren nach Anspruch 1, wobei der zweite Bereich (B) und der dritte Bereich (C) parallel geschaltet sind und mit dem ersten Bereich (A) durch einen fünften Widerstand in Serie geschaltet sind.

4. Verfahren nach einem der vorhergehenden Ansprüche, wobei das Hautimpedanzmodell dargestellt wird als:

$$Z = \frac{R_1 k_1 (j\omega)^{-\alpha 1}}{R_1 + k_1 (j\omega)^{-\alpha 1}} +$$

$$\frac{R_2 R_3 R_4 k_2 (j\omega)^{-\alpha 2} + R_2 (R_3 + R_4) k_2 k_3 (j\omega)^{-(\alpha 2 + \alpha 3)}}{R_2 R_3 R_4 + (R_2 R_3 + R_3 R_4) k_2 (j\omega)^{-\alpha 2} + R_2 (R_3 + R_4) k_2 k_3 (j\omega)^{-\alpha 3} + (R_2 + R_3 + R_4) k_2 k_3 (j\omega)^{-(\alpha 2 + \alpha 3)}}$$

5. Computerprogramm umfassend Computerprogrammcodemittel, die dazu ausgebildet sind, dass das Verfahren nach einem der vorhergehenden Ansprüche ausgeführt wird, wenn das Programm auf einem Computer läuft.

6. Computerprogramm nach Anspruch 5, das auf einem computerlesbaren Medium verkörpert ist.

**Revendications**

1. Procédé d'analyse de l'impédance de la peau d'une partie prédéterminée d'un corps vivant, le procédé comprenant les étapes consistant à :

   appliquer un courant prédéterminé entre une première borne (1) et une troisième borne (3) sur la partie prédéterminée ;
   obtenir des données d'impédance de peau en mesurant une tension entre une deuxième borne (2) sur la partie prédéterminée et la troisième borne (3), la deuxième borne (2) étant située entre la première borne (1) et la troisième borne (3) ; et

fournir un modèle d'impédance de peau entre la deuxième borne (2) et la troisième borne (3) en utilisant les données d'impédance de peau, le modèle comprenant :

une première zone (A) ayant une première résistance et un premier élément à phase constante connectés en parallèle ;
une deuxième zone (B) ayant une deuxième résistance et un deuxième élément à phase constante connectés en parallèle, et une troisième résistance connectée en série à la connexion parallèle de la deuxième résistance et du deuxième élément à phase constante ; et
une troisième zone ayant un troisième élément à phase constante,
dans lequel la deuxième zone et la troisième zone sont connectées en parallèle et sont connectées en série à la première zone.

2. Procédé selon la revendication 1, dans lequel la troisième zone (C) a une quatrième résistance connectée en parallèle au troisième élément à phase constante.

3. Procédé selon la revendication 1, dans lequel la deuxième zone (B) et la troisième zone (C) sont connectées en parallèle et sont connectées en série à la première zone (A) par l'intermédiaire d'une cinquième résistance.

4. Procédé selon l'une quelconque des revendications précédentes, dans lequel le modèle d'impédance de peau est exprimé par :

$$Z = \frac{R_1 k_1 (j\omega)^{-\alpha_1}}{R_1 + k_1 (j\omega)^{-\alpha_1}} + \frac{R_2 R_3 R_4 k_2 (j\omega)^{-\alpha_2} + R_2 (R_3 + R_4) k_2 k_3 (j\omega)^{-(\alpha_2 + \alpha_3)}}{R_2 R_3 R_4 + (R_2 R_3 + R_3 R_4) k_2 (j\omega)^{-\alpha_2} + R_2 (R_3 + R_4) k_3 (j\omega)^{-\alpha_3} + (R_2 + R_3 + R_4) k_2 k_3 (j\omega)^{-(\alpha_2 + \alpha_3)}}.$$

5. Programme informatique comprenant des moyens formant code de programme d'ordinateur adaptés pour mettre en oeuvre le procédé selon l'une quelconque des revendications précédentes lorsque ledit programme est exécuté sur un ordinateur.

6. Programme informatique selon la revendication 5, mis en oeuvre sur un support pouvant être lu par un ordinateur.

# FIG. 1 (PRIOR ART)

# FIG. 2 (PRIOR ART)

## FIG. 3 (PRIOR ART)

## FIG. 4 (PRIOR ART)

## FIG. 5 (PRIOR ART)

## FIG. 6 (PRIOR ART)

## FIG. 7 (PRIOR ART)

## FIG. 8 (PRIOR ART)

## FIG. 9 (PRIOR ART)

## FIG. 10 (PRIOR ART)

# FIG. 11

# FIG. 12

# FIG. 13

| FREQUENCY | RESISTANCE VALUE | REACTANCE VALUE | FREQUENCY | RESISTANCE VALUE | REACTANCE VALUE | FREQUENCY | RESISTANCE VALUE | REACTANCE VALUE | FREQUENCY | RESISTANCE VALUE | REACTANCE VALUE |
|---|---|---|---|---|---|---|---|---|---|---|---|
| 1000 | 1259.3 | -3452.99 | 6946 | 443.2 | -605.90 | 46454 | 337.2 | -149.53 | 310654 | 265.9 | -71.68 |
| 1039 | 1213.1 | -3340.99 | 7216 | 439.8 | -585.94 | 48253 | 335.5 | -146.46 | 322688 | 264.8 | -71.44 |
| 1079 | 1167.8 | -3233.88 | 7495 | 436.6 | -566.71 | 50122 | 333.6 | -143.35 | 335188 | 263.8 | -71.33 |
| 1121 | 1128.2 | -3128.62 | 7785 | 433.6 | -548.27 | 52064 | 331.9 | -140.53 | 348171 | 262.8 | -71.17 |
| 1164 | 1085.8 | -3025.66 | 8087 | 430.5 | -530.29 | 54081 | 330.1 | -137.53 | 361658 | 261.7 | -71.11 |
| 1209 | 1048.0 | -2926.52 | 8400 | 427.6 | -513.17 | 56175 | 328.2 | -134.77 | 375667 | 260.6 | -71.13 |
| 1256 | 1012.8 | -2832.15 | 8726 | 424.8 | -496.61 | 58351 | 326.5 | -132.09 | 390219 | 259.6 | -71.15 |
| 1305 | 979.5 | -2737.56 | 9064 | 422.0 | -480.68 | 60612 | 324.7 | -129.48 | 405335 | 258.6 | -71.28 |
| 1355 | 948.8 | -2646.95 | 9415 | 419.4 | -465.21 | 62960 | 323.0 | -126.86 | 421036 | 257.5 | -71.37 |
| 1408 | 916.9 | -2558.81 | 9779 | 416.5 | -450.35 | 65398 | 321.2 | -124.47 | 437345 | 256.4 | -71.62 |
| 1462 | 889.8 | -2473.54 | 10158 | 414.0 | -436.08 | 67932 | 319.6 | -122.02 | 454286 | 255.3 | -71.85 |
| 1519 | 862.2 | -2391.66 | 10552 | 411.7 | -422.39 | 70563 | 317.8 | -119.74 | 471883 | 254.2 | -72.19 |
| 1578 | 837.7 | -2311.08 | 10960 | 409.3 | -409.04 | 73296 | 316.0 | -117.46 | 490162 | 253.2 | -72.51 |
| 1639 | 813.9 | -2233.85 | 11385 | 407.1 | -396.20 | 76135 | 314.4 | -115.29 | 509149 | 252.0 | -72.95 |
| 1702 | 791.3 | -2158.38 | 11826 | 404.8 | -383.92 | 79085 | 312.7 | -113.13 | 528871 | 250.9 | -73.40 |
| 1768 | 770.1 | -2085.83 | 12284 | 402.6 | -372.15 | 82148 | 311.1 | -111.10 | 549357 | 249.7 | -73.96 |
| 1837 | 750.1 | -2015.72 | 12760 | 400.5 | -360.81 | 85330 | 309.5 | -109.08 | 570637 | 248.5 | -74.47 |
| 1908 | 731.3 | -1947.22 | 13254 | 398.3 | -349.84 | 88636 | 307.8 | -107.17 | 592741 | 247.3 | -75.12 |
| 1982 | 713.0 | -1881.69 | 13768 | 396.1 | -339.25 | 92069 | 306.2 | -105.26 | 615702 | 246.0 | -75.82 |
| 2059 | 696.7 | -1817.67 | 14301 | 394.1 | -329.06 | 95635 | 304.6 | -103.46 | 639551 | 244.9 | -76.47 |
| 2138 | 680.2 | -1756.48 | 14855 | 392.1 | -319.35 | 99340 | 303.1 | -101.60 | 664325 | 243.5 | -77.24 |
| 2221 | 665.4 | -1696.25 | 15430 | 390.3 | -309.99 | 103188 | 301.5 | -99.88 | 690058 | 242.2 | -78.12 |
| 2307 | 650.7 | -1639.18 | 16028 | 388.3 | -300.95 | 107185 | 300.0 | -98.18 | 716788 | 240.8 | -78.98 |
| 2397 | 637.6 | -1583.53 | 16649 | 386.5 | -292.22 | 111337 | 298.5 | -96.63 | 744554 | 239.4 | -79.91 |
| 2490 | 624.7 | -1529.50 | 17294 | 385.2 | -286.85 | 115649 | 297.0 | -95.05 | 773395 | 237.9 | -80.84 |
| 2586 | 612.5 | -1477.43 | 17964 | 383.3 | -278.66 | 120129 | 295.6 | -93.54 | 803353 | 236.4 | -81.91 |
| 2686 | 601.0 | -1427.23 | 18659 | 381.4 | -270.79 | 124783 | 294.2 | -92.05 | 834472 | 234.9 | -82.95 |
| 2790 | 589.9 | -1378.70 | 19382 | 379.6 | -263.29 | 129616 | 292.8 | -90.63 | 866796 | 233.2 | -84.03 |
| 2898 | 580.1 | -1331.82 | 20133 | 377.7 | -256.04 | 134637 | 291.5 | -89.27 | 900372 | 231.6 | -85.17 |
| 3011 | 570.5 | -1286.59 | 20913 | 375.7 | -249.10 | 139852 | 290.1 | -87.91 | 935248 | 229.9 | -86.36 |
| 3127 | 561.4 | -1242.86 | 21723 | 373.9 | -242.42 | 145270 | 288.7 | -86.63 | 971476 | 228.2 | -87.55 |
| 3248 | 552.7 | -1200.59 | 22564 | 372.1 | -235.89 | 150897 | 287.4 | -85.44 | 1009107 | 226.3 | -88.82 |
| 3374 | 544.3 | -1159.63 | 23438 | 370.3 | -229.72 | 156742 | 286.1 | -84.28 | 1048196 | 224.4 | -90.07 |
| 3505 | 536.3 | -1120.23 | 24346 | 368.4 | -223.72 | 162813 | 284.9 | -83.10 | 1088799 | 222.4 | -91.38 |
| 3641 | 529.1 | -1082.20 | 25289 | 366.6 | -217.91 | 169120 | 283.6 | -82.07 | 1130974 | 220.4 | -92.72 |
| 3782 | 522.2 | -1045.54 | 26269 | 364.7 | -212.30 | 175671 | 282.4 | -81.01 | 1174784 | 218.3 | -94.07 |
| 3928 | 515.5 | -1010.19 | 27287 | 363.0 | -207.11 | 182476 | 281.2 | -80.07 | 1220290 | 216.1 | -95.46 |
| 4080 | 508.8 | -975.81 | 28343 | 361.1 | -201.98 | 189544 | 280.0 | -79.12 | 1267559 | 213.8 | -96.92 |
| 4238 | 502.3 | -942.80 | 29441 | 359.4 | -196.90 | 196886 | 278.9 | -78.33 | 1316659 | 211.5 | -98.37 |
| 4403 | 496.4 | -911.01 | 30582 | 357.5 | -192.22 | 204513 | 277.7 | -77.43 | 1367661 | 209.0 | -99.83 |
| 4573 | 491.1 | -880.16 | 31766 | 355.6 | -187.54 | 212435 | 276.7 | -76.66 | 1420639 | 206.5 | -101.20 |
| 4750 | 485.8 | -850.46 | 32997 | 353.8 | -183.18 | 220664 | 275.6 | -75.91 | 1475668 | 203.9 | -102.54 |
| 4934 | 480.8 | -821.83 | 34275 | 352.0 | -178.91 | 229212 | 274.4 | -75.22 | 1532830 | 201.3 | -103.93 |
| 5125 | 475.8 | -794.28 | 35603 | 350.3 | -174.90 | 238090 | 273.3 | -74.66 | 1592205 | 198.5 | -105.28 |
| 5324 | 471.1 | -767.64 | 36982 | 348.2 | -170.84 | 247313 | 272.2 | -74.06 | 1653881 | 195.6 | -106.64 |
| 5530 | 466.6 | -741.98 | 38414 | 346.6 | -166.96 | 256893 | 270.9 | -73.60 | 1717946 | 192.8 | -107.93 |
| 5744 | 462.3 | -717.13 | 39902 | 344.8 | -163.34 | 266844 | 269.9 | -73.07 | 1784492 | 189.8 | -109.23 |
| 5967 | 457.9 | -693.25 | 41448 | 343.0 | -159.59 | 277180 | 269.0 | -72.70 | 1853616 | 186.9 | -110.55 |
| 6198 | 454.0 | -670.25 | 43054 | 341.1 | -156.19 | 287917 | 267.9 | -72.27 | 1925417 | 183.8 | -111.93 |
| 6438 | 450.3 | -648.01 | 44721 | 339.2 | -152.70 | 299070 | 266.9 | -71.97 | 2000000 | 180.4 | -113.25 |
| 6687 | 446.7 | -626.53 | | | | | | | | | |

## FIG. 14

## FIG. 15

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LEE J. W. et al.** Characteristics of Human Skin Impedance Including at Biological Active Points. *IEICE Transactions on Fundamentals of Electronics, Communications and Computer Sciences, Institute of Electronics Information and Comm. Eng.,* June 2003, vol. E86A (6), 1476-1479